# EUROPÄISCHE PATENTSCHRIFT

(11) **EP 0 481 288 B1**
(45) Veröffentlichungstag und Bekanntmachung des Hinweises auf die Patenterteilung: **26.07.1995**
(21) Anmeldenummer: 91116812.8
(22) Anmeldetag: 02.10.1991
(51) Int. Cl.: C07D 211/58, A61K 31/445

(54) **1-Phenylpiperid-4-ylamine und diese enthaltende Arzneimittel**
1-Phenylpiperid-4-ylamines and drugs containing them
1-Phénylpipérid-4-ylamines et médicaments les contenant

(30) Priorität: 16.10.1990 DE 4032767
(43) Veröffentlichungstag der Anmeldung: 22.04.1992
(73) Patentinhaber: BASF Aktiengesellschaft, 67063 Ludwigshafen (DE)
(72) Erfinder: Lubisch, Wilfried, Dr., W-6800 Mannheim 1 (DE); Schult, Sabine, Dr., W-6900 Heidelberg (DE); Binder, Rudolf, Dr., W-6520 Worms (DE); Raschack, Manfred, Dr., W-6714 Weisenheim am Sand (DE); Reinhardt, Roland, Dr., W-6750 Kaiserslautern (DE); Seemann, Dietmar, Dr., W-6907 Nussloch (DE)

(56) Entgegenhaltungen:
- EP-A- 0 291 210
- US-A- 4 902 800
- CHEMISCHE BERICHTE. Bd. 74, Nr. 10, Oktober 1941, WEINHEIM DE Seiten 1648 - 1657; E. CERKOVNIKOV, V. PRELOG: 'Über eine neue Reihe von spasmolytischen Verbindungen. Die substituierten 4-Aminopiperidine, I. Mitteilung'

## Beschreibung

Die Erfindung betrifft 1-Phenylpiperidin-4-ylaminderivate der Formel I und diese enthaltene Arzneimittel, insbesondere zur Verwendung als Antiarrhythmika der Klasse III und als Wirkstoffe mit σ-Rezeptoren-Affinität.

1-Phenylpiperidin-4-ylamine sind beschrieben in BE 678 063 (Protozoenwachstumshemmer) und US 4 902 800 (Interleukin-1-Inhibitoren). Ferner sind 1-Phenylpiperidin-4-ylamine mit antihistaminischer Wirkung beschrieben worden in DRP 749 887 (1941); E. Cerkovnikow et al., Chem. Ber. 74, 1648, 1658 und 1661 (1941) und V. Hahn et al., Helv. Chim. Acta 26, 1132 (1943).

Der Erfindung lag die Aufgabe zugrunde, neue Antiarrhythmika der Klasse III nach Vaughan Williams (Mechanisms and Treatment of Cardiac arrhythmias; Edit. H.J. Reiser und L.N. Horowitz, Verlag Urban und Schwarzenberg, Baltimore und München 1985, Kapitel II.C) mit verbesserten Eigenschaften zu entwickeln.

Die Lösung dieser Aufgabe besteht in 1-Phenylpiperidin-4-ylaminen der Formel I 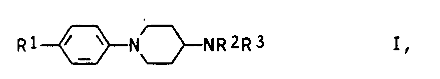
worin
R¹ -NHSO₂R⁴ oder 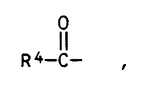
R² und R³ unabhängig voneinander H oder C₁-C₄-Alkyl oder gemeinsam eine Kette (CH₂)ₙ mit n = 4 oder 5 darstellen und
R⁴ C₁-C₄-Alkyl oder Phenyl bedeutet,
sowie deren physiologisch verträglichen Salzen und diese enthaltenden Arzneimitteln.

Die erfindungsgemäßen Verbindungen können nach folgenden Verfahren hergestellt werden: 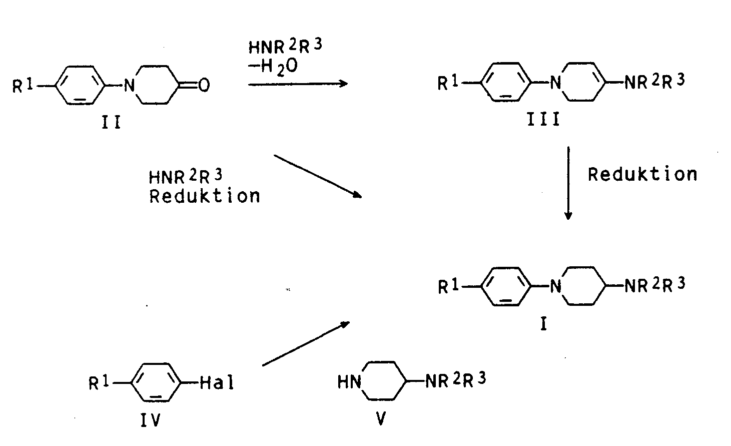
Das Piperidon II (Synthesis 1981, 606-608) wird bei erhöhter Temperatur unter Säure-Katalyse, z.B. mit p-Toluolsulfonsäure und Ameisensäure, in einem aprotischen Lösungsmittel, bevorzugt Toluol, unter Wasserentzug zum Enamin III umgesetzt, das durch Reduktion mit z.B. Natriumborhydrid oder Wasserstoff/Pd/Kohle in üblichen Lösungsmitteln wie Alkoholen in die erfindungsgemäßen Verbindungen I überführt werden kann. Ebenso erhält man I durch direkte Reaktion der Piperidone II mit Aminen HNR²R³, gelöst in üblichen Lösungsmitteln, bevorzugt Alkoholen, unter reduktiven Bedingungen, z.B. in Gegenwart von Natriumcyanborhydrid oder Wasserstoff/Pd/Kohle. Ausgehend vom aromatischen Halogenid IV (Hal = F, Cl, Br) führt der Umsatz mit dem Amin V in bevorzugt polaren Medien wie Alkoholen und Dimethylformamid in Gegenwart von Basen wie Kaliumkarbonat bei erhöhter Temperatur, vorzugsweise 50-150^{o}C, ebenfalls zu I.

Gegebenenfalls werden die so erhaltenen 1-Phenylpiperidin-4-ylamine in das Säureadditionssalz einer physiologisch verträglichen Säure übergeführt. Eine Zusammenstellung üblicher physiologisch verträglicher Säuren kann z.B. aus Fortschritte der Arzneimittelforschung, 1966, Birkhäuser Verlag, Bd. 10, S. 244 bis 285, Deutschland, Schweiz entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Ethanol oder Propanol, oder einem niederen Keton, wie Aceton, Methylethylketon oder Methylisobutylketon, oder einem Ether wie Diethylether, Tetrahydrofuran oder Dioxan erhalten. Zur besseren Kristallabscheidung können Mischungen der genannten Lösungsmittel verwendet werden. Darüber hinaus können pharmazeutisch vertretbare wäßrige Lösungen von Säure-Additionsverbindungen der 1-Phenylpiperidin-4-ylamine der Formel I durch Auflösen der freien Base in einer wäßrigen Säurelösung hergestellt werden.

Die erfindungsgemäßen 1-Phenylpiperidin-4-ylamine stellen Antiarrhythmika der Klasse III dar. Zudem zeigen sie Affinität zum σ-Rezeptor und stellen somit potentielle Antipsychotika, Antikonvulsiva und Neuroprotektiva dar (vgl. F.C. Tortella TIBS 10, 501, f. (1989)). Weiterhin wurde gefunden, daß die Verbindungen den ATP-sensitiven K-Kanal blockieren (vgl. Ann. Rev. Neuroscience 11, 97-118 (1988)).

Die Erfindung betrifft daher auch therapeutische Mittel zur topischen und vor allem systemischen Anwendung, die eine Verbindung der Formel I als Wirkstoff neben üblichen Trägerstoffen und/oder sonstigen galenischen Hilfsmitteln enthalten.

Die Herstellung der therapeutischen Mittel oder Zubereitungen erfolgt mit den üblichen flüssigen oder festen Trägerstoffen oder Verdünnungsmitteln und den üblicherweise verwendeten pharmazeutisch-technischen Hilfsstoffen, entsprechend der gewünschten Applikationsart und mit einer zur Anwendung geeigneten Dosierung, insbesondere mit 7 bis 1750 mg Wirkstoff pro Dosis, in üblicher Weise, beispielsweise durch Vermischen des Wirkstoffes mit den an sich in solchen Präparaten üblichen festen oder flüssigen Träger- und Hilfsstoffen.

Die Mittel können peroral, parenteral oder topisch verabreicht werden. Derartige Zubereitungen sind beispielsweise` Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Lösungen oder Emulsionen und Sprays.

Die therapeutischen Mittel können die erfindungsgemäß zu verwendenden Verbindungen bei lokaler Anwendung in 0,01 bis 1 %iger Konzentration und bei systemischer Anwendung vorzugsweise in einer Einzeldosis von 0,1 bis 25 mg pro kg Körpergewicht enthalten und täglich in einer oder mehreren Dosierungen je nach Art und Schwere der Erkrankungen verabreicht werden.

üblicherweise verwendete pharmazeutisch-technische Hilfsstoffe sind beispielsweise für die lokale Anwendung Alkohole, wie Ethanol, Isopropanol, oxethyliertes Ricinusöl oder oxethyliertes hydriertes Ricinusöl, Polyacrylsäure, Glycerinmonostearat, Paraffinöl, Vaseline, Wollfett, Polyethylenglykol, Polypropylenglykol, Stearat sowie ethoxylierter Fettalkohol, für die systemische Anwendung Milchzucker, Propylenglykol und Ethanol, Stärke, Talk, Poylvinylpyrrolidon. Den Präparaten kann gegebenenfalls ein Antioxidationsmittel, beispielsweise Tocopherol sowie butyliertes Hydroxyanisol oder butyliertes Hydroxytoluol, oder geschmacksverbessernde Zusätze, Stabilisierungsmittel, Emulgiermittel, Bleichmittel usw. zugesetzt werden. Voraussetzung ist, daß alle bei der Herstellung pharmazeutischer Zubereitungen verwendeten Stoffe toxikologisch unbedenklich und mit den verwendeten Wirkstoffen verträglich sind.

### Ausgangsstoffe

### Präparation 1

Zu 5,0 g (22,7 mmol) 1-(4-Nitrophenyl)-4-piperidon, 1,4 g (22,7 mmol) Essigsäure und 3,4 g (45,4 mmol) Diethylamin in 200 ml Methanol wurden bei Raumtemperatur portionsweise 1,4 g (22,7 mmol) Natriumcyanoborhydrid zugegeben. Man ließ alles 16 h rühren und entfernte anschließend das Lösungsmittel im Vakuum. Der Rückstand wurde zwischen Essigsäureethylester und Wasser verteilt, die organische Phase getrocknet und im Vakuum eingeengt. Das so erhaltene Produkt wurde in Aceton aufgenommen und mit etherischer Chlorwasserstoff-Lösung gefällt. Man erhielt 4-(N,N-Diethylamino)-1-(4-nitrophenyl)-piperidinhydrochlorid. Schmp. 183-184°C.

### Präparation 2

5,0 g (22,7 mmol) 1-(4-Nitrophenyl)-4-piperidon, 16,0 g (220 mmol) Diethylamin und 5 ml Ameisensäure in 150 ml Toluol wurden 5 h unter Rückfluß am Wasserabscheider gekocht. Das Lösungsmittel wurde im Vakuum entfernt und 4-(N,N-Diethylamino)-1-(4-nitrophenyl)-1,2,5,6-tetrahydropyridin als Rohprodukt erhalten. Zu diesem Rohprodukt in 150 ml Ethanol wurden bei 10°C 3,45 g (92,5 mmol) Natriumborhydrid portionsweise zugefügt. Man ließ alles 3 h bei Raumtemperatur rühren und entfernte anschließend das Lösungsmittel im Vakuum. Der Rückstand wurde zwischen Wasser und Methylenchlorid verteilt, die organische Phase getrocknet und im Vakuum eingeengt. Dieses Rohprodukt wurde in wenig Isopropanol gelöst und mit etherischer Chlorwasserstofflösung versetzt. Man erhielt 5,5 g des 4-(N,N-Diethylamino)-1-(4-nitrophenyl)-piperidinhydrochlorids, das identisch mit dem Produkt der Präparation 1 war. Schmp. 183-184°C.

### Beispiel 1

5,0 g (20,2 mmol) des Produktes aus Präparation 1 wurde in üblicher Weise an Pd/Kohle in Methanol hydriert. Man erhielt 1-(4-Aminophenyl)-4-(N,N-diethylamino)-piperidin.

Zu 4,4 g (20,2 mmol) dieses Produktes und 1,7 g (21 mmol) Pyridin in 100 ml wasserfreiem Tetrahydrofuran wurden bei 0-5°C 2,4 g (21 mmol) Methansulfonylchlorid, gelöst in 10 ml Tetrahydrofuran, zugetropft. Man ließ 6 h bei Raumtemperatur rühren und trennt das ausgefallene Produkt ab. Man erhielt 4-(N,N-Diethylamino)-1-(4-methansulfonylaminophenyl)-piperidin. Schmp. 214-215°C.

### Beispiel 2

2,1 g (13,25 mmol) 4-(N,N-Diethylamino)piperidin, 1,8 g (13,25 mmol) 4-Fluoracetophenon und 7,3 g Kaliumcarbonat in 100 ml Dimethylformamid/n-Propanol (1:1) wurden 20 h unter Rückfluß gekocht. Man erhielt 1,9 g 1-(4-Acetylphenyl)-4-(N,N-diethylamino)-piperidin, das als Fumarat kristallisierte. Schmp. 125-127°C.

## Patentansprüche

1. 1-Phenylpiperidin-4-ylamine der Formel I 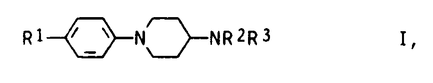 worin
R¹ -NHSO₂R⁴ oder 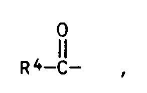 R² und R³ unabhängig voneinander H oder C₁-C₄-Alkyl oder gemeinsam eine Kette (CH₂)ₙ mit n = 4 oder 5 darstellen und
R⁴ C₁-C₄-Alkyl oder Phenyl bedeutet,
sowie deren physiologisch verträgliche Salze.

2. Arzneimittel, das neben üblichen Hilfsstoffen pro Dosis 7 bis 1750 mg einer Verbindung nach Anspruch 1 enthält.

## Claims

1. A 1-phenylpiperidin-4-ylamine of the formula I 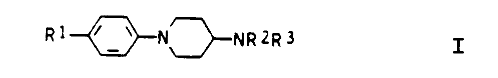 where
R¹ is -NHSO₂R⁴ or 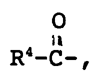 R² and R³ are each, independently of one another, H or C₁-C₄-alkyl or together are a (CH₂)ₙ chain with n = 4 or 5, and
R⁴ is C₁-C₄-alkyl or phenyl,
and the physiologically tolerated salts thereof.

2. A drug which, besides conventional auxiliaries, contains from 7 to 1750 mg of a compound as claimed in claim 1 per dose.

## Revendications

1. 1-Phénylpipérid-4-ylamines de formule I 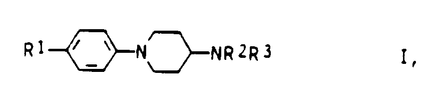 dans laquelle
R¹ représente un groupement -NHSO₂R⁴ ou 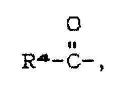
R² et R³ représentent chacun, indépendamment l'un de l'autre, un atome d'hydrogène ou un reste alkyle en C₁-C₄ ou forment ensemble une chaîne (CH₂)ₙ, avec n = 4 ou 5, et
R⁴ représente un reste alkyle en C₁-C₄ ou phényle,
ainsi que leurs sels acceptables physiologiquement.

2. Médicament qui contient par unité posologique, en dehors d'excipients ou d'adjuvants usuels, de 7 à 1750 mg d'un compose selon la revendication 1.
